# EUROPÄISCHE PATENTSCHRIFT

(11) **EP 0 533 790 B1**
(45) Veröffentlichungstag und Bekanntmachung des Hinweises auf die Patenterteilung: **01.10.1997**
(21) Anmeldenummer: 91911459.5
(22) Anmeldetag: 07.06.1991
(51) Int. Cl.: C07D 401/12, A61K 31/415, A61K 31/44

(54) **NEUE SALZFORM VON 5-DIFLUORMETHOXY-2- [3,4-DIMETHOXY-2-PYRIDYL)METHYLSULFINYL]-1H-BENZIMIDAZOL**
NEW SALT FORM OF 5-DIFLUOROMETHOXY-2- [3,4-DIMETHOXY-2-PYRIDYL)METHYLSULFINYL-1H-BENZIMIDAZOLE
NOUVELLE FORME DE SEL DE 5-DIFLUORMETHOXY-2- [3,4-DIMETHOXY-2-PYRIDYLE)METHYLSULFINYL-1H-BENZIMIDAZOLE

(30) Priorität: 11.06.1990 DE 4018642
(43) Veröffentlichungstag der Anmeldung: 31.03.1993
(73) Patentinhaber: BYK GULDEN LOMBERG CHEMISCHE FABRIK GMBH, D-78403 Konstanz (DE)
(72) Erfinder: REITER, Winfried, D-7750 Konstanz (DE); GRÖMMINGER, Konrad, D-7768 Stockach 13 (DE); KOHL, Bernhard, D-7750 Konstanz 19 (DE)
(86) Internationale Anmeldenummer: EP9101057
(87) Internationale Veröffentlichungsnummer: WO9119710

(56) Entgegenhaltungen:
- EP-A- 0 166 287

## Beschreibung

### Anwendungsgebiet der Erfindung

Die Erfindung betrifft die neue Salzform eines bekannten pharmazeutischen Wirkstoffes, die in der pharmazeutischen Industrie zur Herstellung von Arzneimitteln eingesetzt wird.

### Bekannter technischer Hintergrund

Aus dem europäischen Patent 166 287 ist die Verbindung 5-Difluormethoxy-2-[(3,4-dimethoxy-2-pyridyl)methylsulfinyl]-1H-benzimidazol bekannt.

### Beschreibung der Erfindung

Die Erfindung betrifft eine neue Salzform des 5-Difluormethoxy-2-[(3,4-dimethoxy-2-pyridyl)methylsulfinyl]-1H-benzimidazol-Natriumsalzes, die dadurch gekennzeichnet ist, daß sie
- einen Wassergehalt von 4,0-4,5 % aufweist,
- einen Schmelzpunkt von ca. 150-153°C hat,
- schwerlöslich in Aceton ist und
- in Form kubischer Kriställchen kristallisiert.

Die erfindungsgemäße neue Salzform, die aufgrund des Wassergehaltes von 4,0 bis 4,5 % als Monohydrat bezeichnet werden kann, unterscheidet sich von einer weiteren Hydratform (die als 1,5-Hydrat bezeichnet werden kann und die einen Wassergehalt von 6,0-6,5 % aufweist, einen Schmelzpunkt von 137-140° C hat, leichtlöslich in Aceton oder anderen niederen Ketonen ist und in Form feiner Nadeln kristallisiert) in überraschender und vorteilhafter Weise.

Das erfindungsgemäße Monohydrat besitzt in wäßrigem Phosphatpuffer (pH 7,4) bei 22°C eine 5- bis 10-fach höhere Lösegeschwindigkeit als das 1,5-Hydrat. Darüberhinaus weist das Monohydrat - gemessen in Anwesenheit von Bodenkörper - eine etwa doppelt so große Grenzlöslichkeit (13 x 10⁻⁴ mol/l) in Phosphatpuffer (bei 22°C, pH 7,4) auf wie das 1,5-Hydrat (Grenzlöslichkeit 6,2 x 10⁻⁴ mol/l).

Weiterhin erweist sich das Monohydrat überraschenderweise als nicht hygroskopisch. So nimmt es bei Temperaturen von 20 bzw. 30°C und einer relativen Luftfeuchtigkeit von 80 % bei der Lagerung kein Wasser auf (keine Umwandlung in das 1,5-Hydrat!).

Das Verfahren zur Herstellung des Monohydrates, das ebenfalls Gegenstand der Erfindung ist, erweist sich ebenfalls als besonders vorteilhaft, da das Monohydrat durch Vorlegen der freien Verbindung in Aceton oder einem anderen niederen Keton, gegebenenfalls unter Zusatz von Wasser, durch Zugabe von Natronlauge sofort in besonders reiner Form erhalten wird (Auskristallisieren des in Aceton schwer löslichen Monohydrats im Gegensatz zum 1,5-Hydrat, das in Aceton leicht löslich ist). Alternativ kann das Monohydrat durch Auflösen des 1,5-Hydrates in Aceton oder einem anderen niederen Keton, gegebenenfalls unter Zusatz von Wasser, zur Kristallisation gebracht werden.

Als niedere Ketone seien beispielsweise Ethylmethylketon (2-Butanon), Methylisobutylketon (4-Methyl-2-pentanon) oder Diethylketon (3-Pentanon) genannt.

### Beispiele

### 1. 5-Difluormethoxy-2-[(3,4-dimethoxy-2-pyridyl)methylsulfinyl]-1H-benzimidazol-Natriumsalz-monohydrat

10 g (26,08 mMol) 5-Difluormethoxy-2-[(3,4-dimethoxy-2-pyridyl)methylsulfinyl]--1H-benzimidazol werden in 50 ml Aceton unter schwachem Erwärmen gelöst. Durch Zusatz einer Lösung von 1,054 g Natriumhydroxid (26,34 mMol) in 3 bis 30 ml Wasser entsteht die Titelverbindung, welche beim Abkühlen unter Rühren auskristallisiert. Ausbeute: 9,72 g = 92 % d.Th. (Wassergehalt: 4,0 %).

### 2. 5-Difluormethoxy-2-[(3,4-dimethoxy-2-pyridyl)methylsulfinyl]-1H-benzimidazol-Natriumsalz-monohydrat

10 g (26,08 mMol) 5-Difluormethoxy-2-[3,4-dimethoxy-2-pyridyl)methylsulfinyl]-1H-benzimidazol werden in 50 ml Ethylmethylketon unter schwachem Erwärmen gelöst. Durch Zusatz einer Lösung von 1,054 g Natriumhydroxid (26,34 mMol) in 3 bis 30 ml Wasser entsteht die Titelverbindung, welche beim Abkühlen unter Rühren auskristallisiert. Ausbeute: 9,62 g = 91 % d.Th. (Wassergehalt: 4,0 %).

### 3. 5-Difluormethoxy-2-[(3,4-dimethoxy-2-pyridyl)methylsulfinyl]-1H-benzimidazol-Natriumsalz-monohydrat

10 g 5-Difluormethoxy-2-[(3,4-dimethoxy-2-pyridyl)methylsulfinyl]-1H-benzimidazol-Natriumsalz-1,5-hydrat werden unter schwachem Erwärmen in 40 - 80 ml Aceton gelöst. Beim Abkühlen und Rühren der Lösung (ca. 20 Std.) kristallisiert das Monohydrat aus. Ausbeute: 9,10 g = 93,2 % d.Th. (Wassergehalt 4,2 %).

### 4. 5-Difluormethoxy-2-[(3,4-dimethoxy-2-pyridyl)methylsulfinyl]-1H-benzimidazol-Natriumsalz-monohydrat

10 g 5-Difluormethoxy-2-[(3,4-dimethoxy-2-pyridyl)methylsulfinyl]-1H-benzimidazol-Natriumsalz-1,5-hydrat werden unter schwachem Erwärmen in 40 - 80 ml Ethylmethylketon gelöst. Beim Abkühlen und Rühren der Lösung (ca. 20 Std.) kristallisiert das Monohydrat aus. Ausbeute: 9,25 g = 94,9 % d.Th. (Wassergehalt: 4,1 %).

### Gewerbliche Anwendbarkeit

Das erfindungsgemäße Monohydrat stellt eine neue Salzform des 5-Difluormethoxy-2-[(3,4-dimethoxy-2-pyridyl)methylsulfinyl]-1H-benzimidazol dar, die sich in hervorragender Weise sowohl für die Herstellung und Formulierung des Wirkstoffes, als auch für die Anwendung am Patienten eignet.

Bei der Herstellung der neuen Salzform kann auf ein wohlfeiles, wenig toxisches Lösungsmittel (Aceton) zurückgegriffen werden, das gut wieder aufgearbeitet und daher im Kreisprozess wieder eingesetzt werden kann. Das Monohydrat selbst fällt sofort in besonders reiner, kristalliner Form an, die gut abgesaugt und leicht getrocknet werden kann.

Aufgrund des konstanten Wassergehaltes des Monohydrates und aufgrund der Tatsache, daß das Monohydrat nicht hygroskopisch ist, erscheint diese Salzform, durch deren Einsatz ein konstanter Wirkstoffgehalt in besonderer Weise gewährleistet ist, hervorragend geeignet für alle Arzneimittelformulierungen, in denen der Wirkstoff in fester Form vorliegt (z.B. Tabletten, Dragees, Kapseln etc.).

Aufgrund der hohen Lösegeschwindigkeit und der hohen Grenzlöslichkeit ist die neue Salzform besonders gut geeignet für alle Darreichungsformen, bei denen eine schnelle und quantitative Auflösung des Wirkstoffes und somit ein rascher und sicherer Wirkungseintritt gewährleistet sein muß. Besonders geeignet ist die neue Salzform daher für die orale Applikation.

Bei der Humananwendung hat es sich als besonders vorteilhaft erwiesen, die neue Salzform bei der oralen Anwendung in einer Tagesdosis von 10 bis 60 mg, vorzugsweise 20 bis 40 mg {bezogen auf die freie Verbindung 5-Difluormethoxy-2-[(3,4-dimethoxy-2-pyridyl)methylsulfinyl]-1H-benzimidazol} zu verabreichen.

## Patentansprüche

1. Hydrat des 5-Difluormethoxy-2-[(3,4-dimethoxy-2-pyridyl)methylsulfinyl]-1H-benzimidazol-Natriumsalzes, das aufgrund seines Wassergehaltes in etwa als Monohydrat bezeichnet werden kann.

2. Hydrat nach Anspruch 1, dadurch gekennzeichnet, daß der Wassergehalt 4,0 - 4,5 % beträgt.

3. Hydrat nach Anspruch 1, dadurch gekennzeichnet, daß der Schmelzpunkt ca. 150-153°C beträgt.

4. Hydrat nach Anspruch 1, dadurch gekennzeichnet, daß es schwerlöslich in Aceton ist.

5. Hydrat nach Anspruch 1, dadurch gekennzeichnet, daß es in Form kubischer Kriställchen kristallisiert.

6. Verfahren zur Herstellung des Hydrates nach Anspruch 1, dadurch gekennzeichnet, daß man 5-Difluormethoxy-2-[(3,4-dimethoxy-2-pyridyl)methylsulfinyl]-1H-benzimidazol in Aceton oder einem anderen niederen Keton, gegebenenfalls unter Zusatz von Wasser, mit Natronlauge umsetzt und das auskristallisierende Hydrat abtrennt.

7. Verfahren zur Herstellung des Hydrates nach Anspruch 1, dadurch gekennzeichnet, daß man das 5-Difluormethoxy-2-[(3,4-dimethoxy-2-pyridyl)methylsulfinyl]-1H-benzimidazol-Natriumsalz-1,5-hydrat in Aceton oder einem anderen niederen Keton, gegebenenfalls unter Zusatz von Wasser, auflöst und das auskristallisierende Hydrat abtrennt.

## Claims

1. Hydrate of the 5-difluoromethoxy-2-[(3,4-dimethoxy-2-pyridyl)methylsulfinyl]-1H-benzimidazole sodium salt, which on account of its water content can be described roughly as monohydrate.

2. Hydrate according to claim 1, characterized in that the water content amounts to 4.0 to 4.5 %.

3. Hydrate according to claim 1, characterized in that the melting point is about 150 to 153 °C.

4. Hydrate according to claim 1, characterized in that it is sparingly soluble in acetone.

5. Hydrate according to claim 1, characterized in that it crystallizes in the form of small cubic crystals.

6. Process for the preparation of the hydrate according to claim 1, characterized in that 5-difluoromethoxy-2-[(3,4-dimethoxy-2-pyridyl)methylsulfinyl]-1H-benzimidazole is reacted in acetone or another lower ketone, if desired with the addition of water, with sodium hydroxide solution and that the hydrate which crystallizes out is separated off.

7. Process for the preparation of the hydrate according to claim 1, characterized in that 5-difluoromethoxy-2-[(3,4-dimethoxy-2-pyridyl)methylsulfinyl]-1H-benzimidazole sodium salt 1.5 hydrate is dissolved in acetone or another lower ketone, if desired with the addition of water, and that the hydrate which crystallizes out is separated off.

## Revendications

1. Hydrate du sel sodique de 5-difluorométhoxy-2-[(3,4-diméthoxy-2-pyridyl)méthylsulfinyl]-1H-benzimidazole que l'on peut désigner, compte tenu de sa teneur en eau, à peu près comme un monohydrate.

2. Hydrate conforme à la revendication 1, caractérisé en ce que sa teneur en eau est comprise entre 4,0 et 4,5 %.

3. Hydrate conforme à la revendication 1, caractérisé en ce que son point de fusion est d'environ 150 - 153 °C.

4. Hydrate conforme à la revendication 1, caractérisé en ce qu'il est difficilement soluble dans l'acétone.

5. Hydrate conforme à la revendication 1, caractérisé en ce qu'il cristallise sous forme de petits cristaux cubiques.

6. Procédé de préparation de l'hydrate conforme à la revendication 1, caractérisé en ce que l'on fait réagir du 5-difluorométhoxy-2-[(3,4-diméthoxy-2-pyridyl)méthylsulfinyl]-1H-benzimidazole dans de l'acétone ou dans une autre cétone inférieure, éventuellement en présence d'eau, avec de la soude et en ce que l'on isole l'hydrate qui se sépare par cristallisation.

7. Procédé de préparation de l'hydrate conforme à la revendication 1, caractérisé en ce que l'on dissout le 1,5-hydrate du sel sodique de 5-difluorométhoxy-2-[(3,4-diméthoxy-2-pyridyl)méthylsulfinyl]-1H-benzimidazole dans de l'acétone ou dans une autre cétone inférieure, éventuellement en présence d'eau, et en ce que l'on isole l'hydrate qui se sépare par cristallisation.
